# EUROPEAN PATENT SPECIFICATION

(11) **EP 0 790 224 B1**
(45) Date of publication and mention of the grant of the patent: **28.04.1999**
(21) Application number: 97100992.3
(22) Date of filing: 23.01.1997
(51) Int. Cl.: C07C 2/54, C07C 2/62

(54) **Staged alkylation process**
Mehrstufenalkylierungsverfahren
Procédé d'alkylation à plusieurs étapes

(30) Priority: 15.02.1996 DK 16496
(43) Date of publication of application: 20.08.1997
(73) Proprietor: Haldor Topsoe A/S, DK-2800 Lyngby (DK)
(72) Inventor: Hommeltoft, Sven Ivar, 3400 Hillerod (DK)
(74) Representative: Grünecker, Kinkeldey, Stockmair & SchwanhäusserAnwaltssozietät

(56) References cited:
- EP-A- 0 433 954
- EP-A- 0 714 871
- FR-A- 865 161

## Description

The present invention relates to improvements in supported liquid phase alkylation of a hydrocarbon substrate in the presence of a fluorinated sulphonic acid catalyst.

Acid catalyzed alkylation of aliphatic hydrocarbons with olefinic hydrocarbons is a well known process for the preparation of high octane gasoline products. Alkylation of hydrocarbons is conventionally accomplished in liquid phase by mixing paraffins and olefins in the presence of a strong acid catalyst and stirring the mixture until the alkylation reaction is completed.

The usual employed acid catalyst in the industrial alkylation of aliphatic hydrocarbons is concentrated sulphuric acid or anhydrous hydrofluoric acid.

Utilization of fluorinated sulphonic acids, as efficient catalysts during the alkylation of aliphatic hydrocarbons with olefins, has recently been disclosed in US Patent No. 5,220,095. This patent is related to supported liquid phase alkylation of a process stream including a hydrocarbon substrate and an olefinic alkylating agent by contact with a fluorinated sulphonic acid catalyst. The catalyst is applied in one or more fixed bed alkylation reactors with polar contact material, in which there is established on the contact material a reaction zone with the fluorinated sulphonic acid catalyst adsorbed within a confined area of type the contact material. In the reaction zone the process stream is converted at alkylating conditions to a product stream of alkylated hydrocarbons by catalysis of the fluorinated sulphonic acid adsorbed on the contact material.

When carrying out this process in adiabatic manner, the temperature in the alkylation reactor is controlled by recycling typically a part of the reactor effluent containing the alkylated product. Cooling of the process stream with alkylated product containing recycle effluent stream represents, however, a major drawback of this process as presence of alkylated product during the alkylation reaction results in lower alkylate quality Furthermore, the effluent recycle stream represents an additional stream, which reduces the overall process economy

It has now been found that alkylation product quality obtained in a supported liquid phase alkylation process of the type disclosed in US Patent No 5,220,095, may still be improved when carrying out the process in a series of alkylation stages with intermediate addition of alkylating agent between at least two stages.

Through intermediate addition of alkylating agent, the process stream is converted at lower alkylate concentration and cooling by recycling of the alkylated product containing stream may thereby be reduced or completely avoided.

Alkylation of a hydrocarbon feedstock proceeds thereby at a number of subsequent alkylation stages at lower olefin concentration in each stage and with a lower temperature increase during the exothermically alkylation reaction.

Accordingly this invention is a staged process for the liquid phase alkylation of a process stream of a hydrocarbon substrate and an olefinic alkylating agent in presence of an acid catalyst comprising a fluorinated sulphonic acid in at least a first alkylation stage and at least a second alkylation stage connected in series, each reaction stage being carried out in at least a first reactor containing a fixed bed of particulate polar contact material, which process comprises steps of:
(a) establishing a reaction zone on the polar contact material arranged in a first reactor of each process stage, the catalyst being moveable adsorbed on a confined area of the contact material in the reactor;
(b) passing a stream of the hydrocarbon substrate and a first stream of the alkylating agent at alkylating conditions in a first flow direction through the first alkylation stage and withdrawing an effluent stream containing alkylated product and unconverted hydrocarbon substrate from the first alkylation stage;
(c) adding a second stream of the alkylating agent to the effluent stream from the first alkylation stage and passing the mixed stream at alkylating conditions in a first flow direction through the second alkylation stage, and
(d) withdrawing a product stream of alkylated product from the second alkylation stage.

As introductorily mentioned, the invention is based on a supported liquid phase alkylation process as disclosed in US patent No 5,220,095. Similar to the known process the acid alkylation catalyst is in the process according to the present invention continuously adsorbed and desorbed on the contact material by interaction with the process stream. Thus, the reaction zone moves as narrow a zone on the contact material being arranged as fixed bed in each of the alkylation reactors. The migration velocity of the zone is thereby much lower than the velocity of the process stream. The term, "movable adsorbed on a confined area of the contact material" as used herein before and in the following description, refers to the above phenomenon of continuous adsorption and desorption of the catalyst on the contact material.

In operating the invention, hydrocarbon substrate is mixed with a first portion of olefinic alkylating agent and the mixed process stream is introduced into the alkylation reactor in the first process stage. Within the reactor the hydrocarbon substrate is alkylated by reaction with the alkylating agent in presence of the alkylation catalyst adsorbed within the reaction zone as described above.

To the effluent from each reaction stage is added further portions of olefinic alkylating agent and the mixed process stream comprising hydrocarbon substrate, added alkylating agent and alkylated product having been formed in the previous stages is passed to the subsequent process stage. To provide a heat sink for the exothermic alkylation reactions, the effluent stream from each stage may be cooled by heat exchange or flash evaporation of part of unconverted hydrocarbon substrate prior to addition of alkylating agent. Typically, flashed hydrocarbon substrate will be recycled to the inlet of the alkylation reactors.

By passage through the alkylation stages with intermediate addition of alkylating agent, the concentration of alkylated product increases continuously to a final level in the last alkylation stage, which substantially corresponds to the alkylate concentration in process of the aforementioned US Patent No. 5,220,095, wherein alkylation of the hydrocarbon substrate is performed with the total amount of olefinic alkylating agent in a single alkylation step optionally with recycle of alkylate effluent stream.

Alkylated product is recovered from the effluent stream from the final alkylation stage by fractionating e.g. distillation.

Unconverted hydrocarbon substrate being separated from the product stream is typically recycled to the inlet of the first alkylation step.

A hydrocarbon substrate for use in the invention comprises paraffins, preferably C₄-Cᵢₒ isoalkanes and/or C₆-C₂₀ aromatic hydrocarbons being alkylated with an olefinic alkylating agent, usually C₂-C₂₀ olefins in a mole ratio of the hydrocarbon substrate and the olefinic alkylating agent of between 1 and 50.

Suitable acid catalysts for use in the invention are fluorinated alkane sulphonic acids, including C₁-C₄ perfluorinated alkane sulphonic acids or mixtures thereof.

A preferred fluorinated sulphonic acid is trifluoromethanesulphonic acid.

Convenient contact materials are any of the polar and non-basic refractory materials. Preferred materials are silica, zirconia, titania, the oxides of tin and the lanthanides or mixtures thereof.

Useful alkylating conditions comprise reaction temperatures of between -40° and 100°C, and a pressure varying in the range of between 1-100 bar abs. depending on the composition of the process stream and the actual reaction temperature employed.

[0021 ] The invention may further be carried out in a series of stages with one alkylation reactor in each stage, wherein each reactor is operated in such way that the migrating reaction zone is cycled between the opposing end of each of the reactors by periodically reversing the flow direction of the process stream.

Accordingly, a further embodiment of the invention comprises the further steps of
(e) interrupting step (b) and step (c);
(f) passing a stream of the hydrocarbon substrate and the first stream of the alkylating agent at alkylating condition in a second flow direction through the first reactor and withdrawing an effluent stream of partly alkylated product from the first reactor; and
(g) passing the effluent stream from the first reactor and a second stream of the alkylating agent at alkylating condition in a second flow direction through the second reactor and withdrawing a product stream of alkylated hydrocarbon substrate from the second reactor.

Alternatively, each reaction stage is carried out in at least two reactors as described for a single stage process in US patent No. 5,245,100. Thereby, the catalyst is cycled between the reactors in each stage.

The alkylation catalyst in each alkylation stage is then passed from the outlet end of a first reactor to a subsequent reactor and moveable adsorbed at the contact material at the inlet end of the second reactor.

Still in an embodiment, the process is carried out in at least a first and second reactor each being provided with a reaction zone of the alkylation catalyst and a third reactor without a reaction zone. Between the first and second reactor additional olefinic alkylating agent is added to the effluent stream from the reactors.

In the later process, the catalyst is passed subsequently from the outlet end of a first reactor to the inlet end of a subsequent reactor without a reaction zone. By this process sequence, the alkylation catalyst is sequentially passed through the reactors.

After the catalyst has been cycled to the subsequent reactor, the first reactor may optionally be flushed with a stream of the hydrocarbon substrate in order to transfer unreacted amounts of alkylating agent together with hydrocarbon substrate to the subsequent reactor.

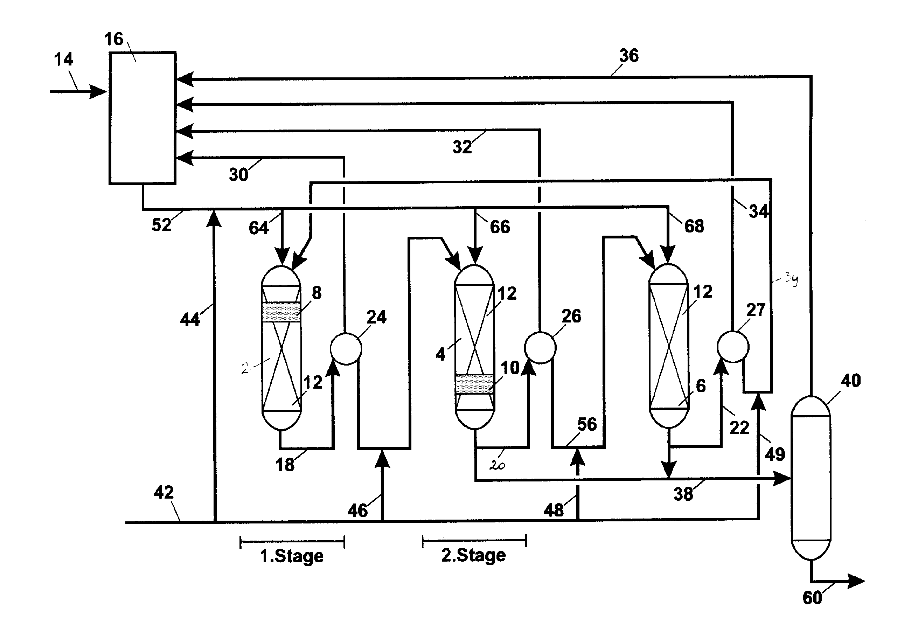

The above features and aspects of the invention will become more apparent from the following detailed description with reference to the drawings in which the sole Figure represents a simplified process scheme of an alkylation process according to a specific embodiment of the invention.

Referring to the Figure, a staged alkylation process is carried out in three reactors connected in series. Reactor 2 and 4 are provided with reaction zone 8 and 10, respectively having a fluorinated sulphonic acid alkylation catalyst absorbed on contact material 12 loaded in the reactors. Reactor 6 is at the process cycle shown in the Figure not provided with a reaction zone on contact material 12.

A feed stream of hydrocarbon substrate 14 is in feed container 16 admixed with unconverted hydrocarbon substrate being flashed off from effluent streams 18, 20 in flash evaporators 24, 26 and recycled to container 16 in lines 30, 32. Unconverted hydrocarbon substrate separated from alkylate product in fractionator 40 is recycled to container 16 in line 36. The combined feed stream is passed to the first alkylation stage, which in the shown process cycle is carried out in reactor 2. Prior to introduction of the feed stream, a first portion of alkylating agent 42 is added through line 44 to the feed stream being passed to reactor 2 in line 52 via line 64. In reactor 2, the alkylating agent reacts with the hydrocarbon substrate by contact with the alkylation catalyst in reaction zone 8. Reaction zone 8 migrates continuously to the outlet end of the reactor, as described above more detailed. Effluent stream 18 containing unconverted hydrocarbon substrate and alkylated product is withdrawn from reactor 2. A part of the substrate is separated in flash evaporator 24 and recycled in line 30 to feed container 16. By evaporation of part of the hydrocarbon substrate effluent stream 18 is cooled.

To the cooled effluent stream a further portion of the alkylating agent is added from line 46 and the mixed stream is introduced into the second process stage, which in the shown process cycle is carried out in reactor 4. In reactor 4, alkylation of the hydrocarbon substrate proceeds in similar manner as in the first process stage. Effluent stream 20 from reactor 4 containing alkylate product and small amounts of unconverted hydrocarbon substrate is passed via evaporator 26, line 56 and reactor 6 to product line 38 and to fractionator 40, wherein the alkylate product obtained by conversion of the substrate in the first and second process stage is recovered by fractionation, e.g. distillation. Alkylate product is then removed in product line 60. Separated hydrocarbon substrate is recycled from fractionator 40 in line 36 to container 16.

As described herein before, reaction zones 8 and 10 continuously move by interaction with the process stream towards the outlet end of the reactors, and are finally transferred with the process stream to a subsequent reactor.

In the shown process scheme, reaction zone 10 in reactor 4 is closer to the reactor outlet than reaction zone 8 in reactor 2.

At the end of the process cycle described above, the alkylation catalyst is transferred from reactor 4 to subsequent reactor 6, wherein a reaction zone (not shown) established with the transferred catalyst adsorbed on contact material 12 in this reactor.

Thus, in a second process cycle, reaction zone 8 is still in reactor 2, whereas a reaction zone has been established in reactor 6. Reactor 4 contains in this cycle no reaction zone on contact material 12. In the second cycle, the first process stage is carried out in reactor 6, while the second stage is carried out in reactor 6.

The feed stream is introduced into reactor 6 through lines 52 and 68 after alkylating agent has been added from line 44 to the stream. The effluent stream from reactor 6, in which this cycle in the first process stage is carried out, is withdrawn from the reactor through line 22 and passed to evaporator 27. A part of unconverted hydrocarbon substrate being removed in evaporator 27 is recycled through line 34 to container 16.

To the cooled effluent stream from evaporator 27 part of the alkylating agent is added from line 49. The mixed stream is then passed through line 39 to reactor 2, in which the second process stage is carried out.

Effluent stream from reactor 2 is passed through reactor 4 to product line 38 and fractionator 40 and treated in similar manner as in the previous process cycle.

In a third process cycle, alkylation catalyst in reaction zone 8 having moved to the outlet end of reactor 2 during the previous cycle is transferred from reactor 2 to reactor 4 together with the effluent stream in line 18. After the catalyst has been transferred to reactor 4, the hydrocarbon feedstream in line 52 is passed via line 66 to the inlet of reactor 4. Prior to introduction into the reactor, the feedstream is admixed with alkylating agent from line 44. Similar to the previous process cycles, effluent 20 from reactor 4, which in the actual process cycle represents the first alkylation stage, is cooled by flash evaporation of part of unreacted hydrocarbon substrate in evaporator 26 and thereafter admixed with a second portion of alkylating agent from line 48. The second alkylation stage is in this process cycle performed in reactor 6 in the same manner as in the previous cycle.

In a fourth process cycle, the alkylation catalyst is transferred from reactor 6 with the effluent stream in line 22 and line 39 to reactor 2 from which the alkylation catalyst has been removed in the previous cycle.

In the fourth cycle, the first process stage is thus carried out in reactor 2 and the second stage in reactor 4 in similar manner as described above.

### Examples

In the following Examples, alkylation is carried out in a single stage in one reactor, Example 1, according to known principles. In Example 2, alkylation is performed in a single cycle similar to the one of Example 1, with the exception that part of the effluent from the alkylation reactor was recycled.

In Example 3 alkylation was carried out in a two stage process with intermediate addition of alkylating agent between the stages according to the invention.

### Example 1

A mixture of 10% by volume of 2-butene in isobutane was introduced at a rate of 0.24 kg/hr into a reactor consisting of a 6 m tube with a cross sectional area of 16 mm² packed with silicagel (Merck 100, 0.2-0.5 mm) contact material. On the material, a catalyst zone was established by adsorption of 6 ml trifluoromethanesulphonic acid. The reactor was submerged into a cooling bath for temperature control. Cg+ alkylate product withdrawn from the reactor was separated from unreacted isobutane by distillation of the obtained product mixture and analyzed by gas chromatography(GC). Octane number RON and MON were estimated based on the analyzed product composition. At a bath temperature of 10°C, an alkylate product with a calculated RON/MON of 95.0/93.0 was produced at a rate of about 52 g/hr. The product composition obtained is summarized in Table 1 below.

### Example 2

A mixture of 10% 2-butene in isobutane was introduced at a rate of 0.24 kg/hr into a reactor consisting of a 6 m tube with a cross sectional area of 16 mm² packed with silicagel (Merck 100, 0.2-0.5 mm) contact material on which a catalyst zone with 6 ml trifluoromethanesulphonic acid has been established. The reactor was submerged into a cooling bath for temperature control. 50% by volume of the reactor effluent were recycled to the reactor inlet giving an olefin (2-butene) concentration at reactor inlet of 5% by volume and a total flow through the reactor of 0.48 kg/hr. Cₛ+ alkylate obtained was separated from unreacted isobutane by distillation of the product mixture and analyzed by GC. Octane number RON and MON were estimated based on the analyzed product composition. At a bath temperature of 10°C, an alkylate product with RON/MON of 95.8/93.6 was produced at a rate of about 52 g/hr. The product composition obtained is summarized in Table 1.

### Example 3

In this Example, two reactors similar to the reactor described in the above Examples without effluent recycle were coupled in series. A mixture of 0.325 kg/hr isobutane and 0.017 kg/hr 2-butene was fed to the first reactor (total flow = 0.342 kg/hr 5% by volume 2-butene). The effluent from this first reactor was mixed with an additional olefin stream (20% w/w) 2-butene, 0.12 kg/hr) and fed to the second reactor. The effluent from the second reactor 0.462 kg/hr contained 87 g/hr C5+ alkylate with RON/MON of 96.4/94.1.

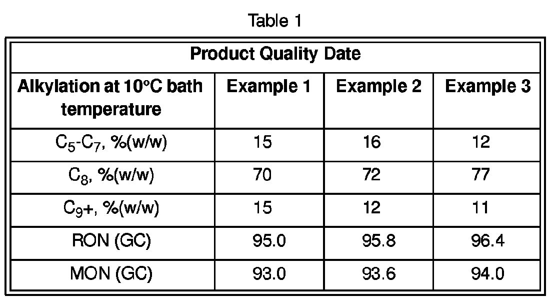

## Claims

1. Process for the liquid phase alkylation of a process stream of a hydrocarbon substrate and an olefinic alkylating agent in presence of an acid catalyst comprising one or more fluorinated sulphonic acids in at least a first alkylation stage and at least a second alkylation stage connected in series, each reaction stage being carried out in at least a first reactor containing a fixed bed of particulate polar contact material, which process comprises steps of:
(a) establishing a reaction zone on the polar contact material arranged in a first reactor of each process stage, the alkylation catalyst being moveable adsorbed on a confined area of the contact material in the reactor;
(b) passing a stream of the hydrocarbon substrate and a first stream of the alkylating agent at alkylating conditions in a first flow direction through the first alkylation stage and withdrawing an effluent stream containing alkylated product and unconverted hydrocarbon substrate from the first alkylation stage;
(c) adding a second stream of the alkylating agent to the effluent stream from the first alkylation stage and passing the mixed stream at alkylating conditions in a first flow direction through the second alkylation stage, and
(d) withdrawing a product stream of alkylated product from the second alkylation stage.

2. The process of claim 1 comprising the further steps of
(e) interrupting step (b) and step (c);
(f) passing a stream of the hydrocarbon substrate and the first stream of the alkylating agent at alkylating condition in a second flow direction through the first reactor and withdrawing an effluent stream of partly alkylated product from the first reactor; and
(g) passing the effluent stream from the first reactor and a second stream of the alkylating agent at alkylating condition in a second flow direction through the second reactor and withdrawing a product stream of alkylated hydrocarbon substrate from the second reactor.

3. The process of claim 1, wherein the reaction zone being established in each alkylation stage is independently cycled between at least two reactors in each alkylation stage.

4. The process of claim 1, wherein alkylation is carried out in at least a first (2) and second reactor (4) each being provided with a reaction zone of the catalyst and a third reactor (6) without a reaction zone, the alkylation catalyst is transferred from outlet of the first reactor to inlet of the second reactor and from outlet of the second reactor to inlet of the third reactor.

5. The process of claim 1, wherein the fluorinated sulphonic acid is selected from the group of CnF₂ₙ,ₗSO₃H sulphonic acids, where n is an integer of 1-4 and mixtures thereof.

6. The process of claim 1, wherein the fluorinated sulphonic acid is trifluoromethanesulphonic acid.

## Patentansprüche

1. Verfahren zur Flüssigphasenalkylierung eines Verfahrensstroms eines Kohlenwasserstoffsubstrats und eines olefinischen Alkylierungsmittels in Gegenwart eines sauren Katalysators, der eine oder mehrere fluorierte Sulfonsäuren umfaßt, in mindestens einem ersten Alkylierungsschritt und mindestens einem zweiten Alkylierungsschritt, die in Reihe miteinander verbunden sind, wobei jeder Reaktionsschritt in mindestens einem ersten Reaktor ausgeführt wird, der ein Festbett von aus einzelnen Teilchen bestehendem, polaren Kontaktmaterial enthält, wobei das Verfahren die folgenden Schritte umfaßt:
(a) das Einrichten einer Reaktionszone auf dem polaren Kontaktmaterial, das in einem ersten Reaktor jedes Verfahrensschritts angeordnet ist, wobei der Alkylierungskatalysator auf einem begrenzten Bereich des Kontaktmaterials im Reaktor beweglich adsorbiert ist;
(b) den Durchgang eines Stroms des Kohlenwasserstoffsubstrats und eines ersten Stroms des Alkylierungsmittels unter Alkylierungsbedingungen in einer ersten Strömungsrichtung durch den ersten Alkylierungsschritt und das Entfernen eines ausströmenden Stroms, der das alkylierte Produkt und das nichtumgesetzte Kohlenwasserstoffsubstrat enthält, vom ersten Alkylierungsschritt;
(c) das Hinzufügen eines zweiten Stroms des Alkylierungsmittels zum vom ersten Alkylierungsschritt ausströmenden Strom und den Durchgang des gemischten Stroms unter Alkylierungsbedingungen in einer ersten Strömungsrichtung durch den zweiten Alkylierungsschritt und
(d) das Entfernen eines Produktstroms des alkylierten Produkts vom zweiten Alkylierungsschritt.

2. Verfahren nach Anspruch 1, das die folgenden weiteren Schritte umfaßt:
(e) das Unterbrechen der Schritte (b) und (c);
(f) den Durchgang eines Stroms des Kohlenwasserstoffsubstrats und des ersten Stroms des Alkylierungsmittels in einer zweiten Strömungsrichtung unter Alkylierungsbedingungen durch den ersten Reaktor und das Entfernen eines ausströmenden Stroms von teilweise alkyliertem Produkt aus dem ersten Reaktor und
(g) den Durchgang des vom ersten Reaktor ausströmenden Stroms und eines zweiten Stroms des Alkylierungsmittels unter Alkylierungsbedingungen in einer zweiten Strömungsrichtung durch den zweiten Reaktor und das Entfernen eines Produktstroms des alkylierten Kohlenwasserstoffsubstrats vom zweiten Reaktor.

3. Verfahren nach Anspruch 1, wobei die Reaktionszone, die in jedem Alkylierungsschritt eingerichtet wird, in jedem Alkylierungsschritt unabhängig zwischen mindestens zwei Reaktoren zykliert.

4. Verfahren nach Anspruch 1, wobei die Alkylierung in mindestens einem ersten Reaktor (2) und zweiten Reaktor (4), die jeweils mit einer Reaktionszone des Katalysators ausgestattet sind, und einem dritten Reaktor (6) ohne eine Reaktionszone ausgeführt wird, wobei der Alkylierungskatalysator vom Auslaß des ersten Reaktors zum Einlaß des zweiten Reaktors und vom Auslaß des zweiten Reaktors zum Einlaß des dritten Reaktors überführt wird.

5. Verfahren nach Anspruch 1, wobei die fluorierte Sulfonsäure aus der Gruppe von 0,F2"lSO3H-Sulfonsäuren, wobei n eine ganze Zahl von 1 bis 4 ist, und Gemischen davon ausgewählt ist.

6. Verfahren nach Anspruch 1, wobei die fluorierte Sulfonsäure Trifluormethansulfonsäure ist.

## Revendications

1. Procédé pour l'alkylation en phase liquide d'un courant de traitement d'un substrat hydrocarbure et d'un agent d'alkylation oléfinique en présence d'un catalyseur acide comprenant un ou plusieurs acides sulfoniques fluorés dans au moins une première étape d'alkylation et au moins une deuxième étape d'alkylation connectées en série, chaque étape de réaction étant effectuée dans au moins un premier réacteur contenant un lit fixe de matériau de contact polaire particulaire, lequel procédé comprenant les étapes consistant à :
(a) établir une zone de réaction sur le matériau de contact polaire disposé dans un premier réacteur de chaque étape de traitement, le catalyseur d'alkylation étant adsorbé de manière déplaçable sur une zone confinée du matériau de contact dans le réacteur ;
(b) faire passer un courant du substrat hydrocarbure et un premier courant de l'agent d'alkylation dans des conditions d'alkylation dans une première direction d'écoulement dans la première étape d'alkylation et retirer un courant d'effluent contenant un produit alkylé et un substrat hydrocarbure non converti de la première étape d'alkylation ;
(c) ajouter un second courant de l'agent d'alkylation au courant d'effluent à partir de la première étape d'alkylation et faire passer le courant mixte dans des conditions d'alkylation dans une première direction d'écoulement dans la seconde étape d'alkylation, et
(d) retirer un courant de produit alkylé de la seconde étape d'alkylation.

2. Procédé selon la revendication 1, comprenant les étapes supplémentaires consistant à :
(e) interrompre l'étape (b) et l'étape (c) ;
(f) faire passer un courant du substrat hydrocarbure et le premier courant de l'agent d'alkylation dans des conditions d'alkylation dans une seconde direction d'écoulement dans le premier réacteur et retirer un courant d'effluent de produit partiellement alkylé du premier réacteur ; et
(g) faire passer le courant d'effluent du premier réacteur et un second courant de l'agent d'alkylation dans des conditions d'alkylation dans une seconde direction d'écoulement dans le second réacteur et retirer un courant de produit de substrat hydrocarbure alkylé du second réacteur.

3. Procédé selon la revendication 1, dans lequel la zone de réaction établie dans chaque étape d'alkylation suit un cycle indépendant entre au moins deux réacteurs dans chaque étape d'alkylation.

4. Procédé selon la revendication 1, dans lequel l'alkylation est effectuée dans au moins un premier (2) et un deuxième réacteurs (4), chacun étant pourvu d'une zone de réaction du catalyseur, et dans un troisième réacteur (6) sans zone de réaction, le catalyseur d'alkylation étant transféré de l'extrémité de sortie du premier réacteur à l'entrée du deuxième réacteur, et depuis l'extrémité de sortie du deuxième réacteur à l'entrée du troisième réacteur.

5. Procédé selon la revendication 1, dans lequel l'acide sulfonique fluoré est choisi dans le groupe composé des acides sulfoniques de type CnF₂ₙ,ₗSO₃H où n est un entier de 1 à 4 et des mélanges de ceux-ci.

6. Procédé selon la revendication 1, dans lequel l'acide sulfonique fluoré est l'acide trifluorométhanesulfonique.
